# EUROPÄISCHE PATENTSCHRIFT

(11) **EP 0 844 855 B1**
(45) Veröffentlichungstag und Bekanntmachung des Hinweises auf die Patenterteilung: **24.10.2001**
(21) Anmeldenummer: 96928429.8
(22) Anmeldetag: 08.08.1996
(51) Int. Cl.: A61F 2/36, A61F 2/30

(54) **ENDOPROTHESE, INSBESONDERE HÜFTGELENKPROTHESE**
ENDOPROSTHESIS, IN PARTICULAR AN ARTIFICIAL HIP JOINT
ENDOPROTHESE, NOTAMMENT PROTHESE DE L'ARTICULATION DE LA HANCHE

(30) Priorität: 16.08.1995 DE 19529988; 19.08.1995 DE 19530531
(43) Veröffentlichungstag der Anmeldung: 03.06.1998
(73) Patentinhaber: Lampe, Frank, 20251 Hamburg (DE); Nassutt, Roman, 23879 Mölln (DE)
(72) Erfinder: Lampe, Frank, 20251 Hamburg (DE); Nassutt, Roman, 23879 Mölln (DE)
(74) Vertreter: von Raffay, Vincenz, Dipl.-Ing.
(86) Internationale Anmeldenummer: EP9603506
(87) Internationale Veröffentlichungsnummer: WO9706752

(56) Entgegenhaltungen:
- EP-A- 0 163 121
- EP-A- 0 176 711
- EP-A- 0 359 485
- EP-A- 0 603 976
- DE-U- 8 903 850
- US-A- 3 833 002

## Beschreibung

Die Erfindung betrifft eine Hüftgelenkprothese nach dem Oberbegriff des Anspruches 1.

Eine derartige Endoprothese ist aus der DE-U-89 03 850 bekannt.

Gelenkendoprothesen ersetzen durch degenerative, entzündliche, traumatische oder tumoröse Veränderungen geschädigte Gelenke und ermöglichen es dem Patienten so, ein weitgehend "normales" Leben zu führen.

Es ist bekannt, daß das ideale Gelenkimplantat eine physiologische, proximal-metaphysäre Lastübertragung in das tragende Knochenlager ermöglichen sollte. Für eine ausreichende Fixierung, insbesondere zur Primärverankerung von zementfreien Implantaten, besitzen die üblichen Implantate vor allem im Hüftbereich einen weit in den Markraum des tragenden Knochens hineinreichenden Prothesenstiel.

Es ist weiterhin bekannt, daß durch die herkömmlichen langstieligen Prothesen die geforderte physiologische Lasteinleitung derart geändert wird, daß anstelle der proximal-metaphysären die distal diaphysäre Krafteinleitung begünstigt wird (Menge, M., 1994, Die metaphysäre Prothesenverankerung - ein neues Konzept für die Femurprothese). Durch diese Kraftumleitung in den distalen Bereich des tragenden Knochens (Femur) entsteht eine mechanische Schonung des Knochens im proximalen Bereich, das "Stress-Shielding". Die Folge ist die oft zu beobachtende Resorption des Knochens im proximalen Bereich, vor allem des Kalkar (Kalkaratrophie). Zusätzlich kommt es distal im Bereich der Stielspitze zu einer verstärkten Krafteinleitung mit resultierender Kortikalishypertrophie (Cohen, C., Rushton, N., 1995, Bone Remodelling in the Proximal Femur After Charnley THA), (Kuiper, J.H., 1993, Three-Dimensional Optimization of Hip Prosthetic Design). Diese Mechanismen führen zur Lockerung der Prothese im Knochenlager und somit zum Versagen des Implantates.

Es ist weiterhin bekannt, daß der Knochensubstanzverlust im Falle einer Wechseloperation die Fixation der Wechselprothese erheblich erschwert und häufig die Verwendung von teuren Sonderimplantaten erfordert.

Es ist weiterhin bekannt, daß sich als bioresorbierbare Materialien bekannte Copolymere verwenden lassen, die z.B. aus D, L-Lactid oder L-Lactid oder aus Glycolid oder aus einer Mischung dieser Stoffe hergestellt werden. Außerdem sind eigenverstärkte Materialien entwickelt worden, die bezüglich ihrer mechanischen Eigenschaften den bisherigen weit überlegen sind.
Diese kommen bereits bei der Herstellung von Knochenschrauben, -stiften und -platten zur Anwendung. Vergleiche z.B. Törmälä, P; 1993, Ultra-high Strength, Self-Reinforced Absorbable Polymeric Composites for Applications in Different Disciplines of Surgery, Clin. Mat. pp. 35-40.

Es ist weiterhin bekannt, daß die Resorption zu großer Mengen bioresorbierbaren Materials im Körper zu unerwünschten Fremdkörperreaktionen führen kann.

Der Erfindung liegt die Aufgabe zugrunde, die eingangs genannte Endoprothese derart fortzubilden, daß die Zahl der belastenden lockerungsbedingten Prothesenwechseloperationen minimiert wird.

Diese Aufgabe wird grundsätzlich durch das Kennzeichen des Anspruches 1 gelöst.

Eine besonders vorteilhafte Ausführungsform ist Gegenstand des Anspruches 2.

Die mit der Erfindung erzielten Vorteile bestehen darin, daß dieser Prothesentyp unmittelbar postoperativ durch seinen langen Stiel eine ausreichende, den heutigen Prothesenvarianten entsprechende Primärstabilität gewährleistet. Durch den in den folgenden Wochen nach der Operation ablaufenden Abbau des bioresorbierbaren Zwischenstückes wird eine stufenlose mechanische Entkopplung des distalen Stielteiles vom proximalen Stielteil erreicht, die eine mechanische Entlastung des distalen Stielteiles zur Folge hat. Gleichzeitig kommt es zum knöchernen Einwachsen des proximalen Stielteiles. Dieser Prozess führt dann zu einer ausschließlich proximalen Verankerung und somit zu einer physiologischen Lasteinleitung in das Knochenlager. Die aseptische Implantatlockerung, die durch mechanisch bedingte Knochenresorptionsvorgänge ausgelöst wird, kann somit vermieden werden. So wird eine deutlich erhöhte Standzeit der Prothese möglich.

Im Falle einer aus anderen Gründen trotzdem notwendigen Wechseloperation wird durch den Erhalt der proximalen Knochensubstanz eine solide Ausgangsposition für die Fixation der Wechselprothese geschaffen.

Die hier beschriebenen Funktionen der neuen Hüftendoprothese werden mit einem kleinen Materialvolumen des Zwischenstückes erreicht. Dies ist deshalb von entscheidender Bedeutung, da größere Mengen bioresorbierbaren Materials im Körper zu unerwünschten Gewebereaktionen führen können.

Bei der Ausführungsform nach Anspruch 2 ist zusätzlich zu dem aus bioresorbierbarem Material bestehenden Verbindungselement ein zusätzliches, aus nicht-resorbierbarem Material, beispielsweise aus Edelstahl, hergestelltes Verbindungselement vorgesehen, das für eine mechanische Stütze sorgt. Dieses zusätzliche Verbindungselement überträgt aber keine Biegekräfte.

Im folgenden wird die Erfindung unter Hinweis auf die Zeichnung anhand eines Ausführungsbeispieles näher erläutert.

### Es zeigt:

- Fig. 1: einen Schnitt durch eine erste Ausführungsform einer erfindungsgemäßen Hüftgelenkprothese; und
- Fig. 2: einen Schnitt durch eine andere Ausführungsform einer erfindungsgemäßen Hüftgelenkprothese.

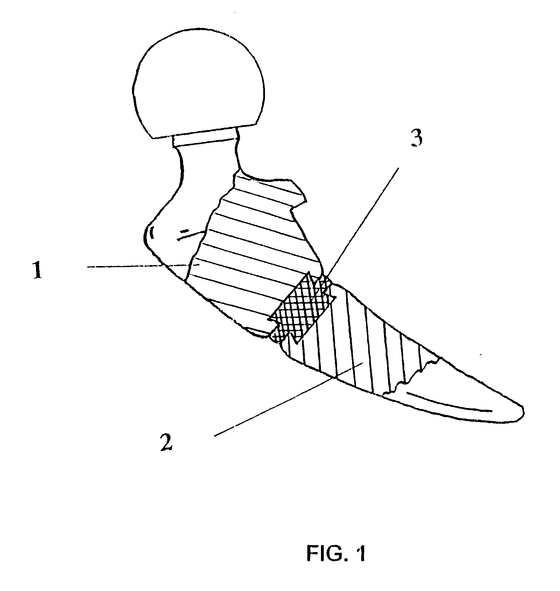

Die in Fig. 1 dargestellte Hüftgelenkprothese besteht aus einem oberen Teil 1 und einem unteren Teil 2. Zwischen diesen beiden Teilen 1 und 2 ist ein aus bioresobierbarem Material gefertigtes Verbindungselement 3 angeordnet.

Durch diese Hüftgelenkprothese wird einer möglichen Lockerung durch Knochenumbauvorgänge entgegengewirkt. Durch Abbau des bioresorbierbaren Zwischenstücks in dem Prothesenstiel kommt es zu einer stufenlosen, mechanischen Entkoppelung des distalen Stielteils vom proximalen Stielteil. So wird eine mechanische Entlastung des distalen Stielteils und damit eine natürliche Lasteinleitung, lediglich über das knöchern eingewachsene proximale Stielteil, in den Knochen erreicht. Die unmittelbar nach der Operation erforderliche Primärstabilität wird dabei trotzdem gewährleistet. Ein Einsatz ist grundsätzlich nicht nur als Hüftgelenkprothese möglich, sondern in allen Röhrenknochen, z.B. Knie, Ellenbogen und Finger.

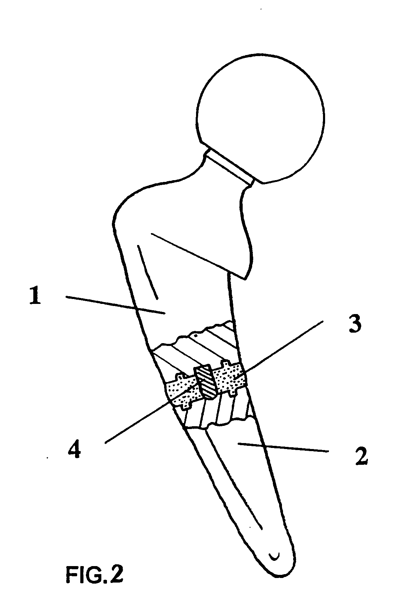

Bei der Ausführungsform nach Fig. 2 ist in dem Bereich des bioresorbierbaren Verbindungselementes 3 ein zusätzliches Verbindungselement 4 aus nicht-resorbierbarem Material, beispielsweise aus Edelstahl, vorgesehen. Dieses zusätzliche Verbindungselement 4 ist so angeordnet und ausgebildet, daß es keine Biegekräfte übertragen kann, aber für eine zusätzliche Aufnahme von Druckkräften in axialer Richtung sorgt. Es kann in dem Oberteil 1 des Schaftes befestigt sein und in dem Unterteil 2 nach Art einer Pfanne "gelenkig" abgestützt sein. Andere Befestigungsarten sind möglich.

## Patentansprüche

1. Endoprothese, insbesondere Hüftgelenkprothese, bestehend aus einem Stiel und einem Gelenkteil, wobei der obere Stielteil (1) und der untere Stielteil (2) mittels eines Verbindungselementes (3) verbunden sind, **dadurch gekennzeichnet, daß** das Verbindungselement (3) aus einem bioresorbierbarem Material gefertigt ist.

2. Endoprothese nach Anspruch 1, **dadurch gekennzeichnet, dass** ein zweites Verbindungselement (4) aus nicht-resorbierbarem Material, beispielsweise Edelstahl, in dem Bereich des ersten Verbindungselementes (3) aus bioresorbierbarem Material angeordnet ist.

3. Endoprothese nach Anspruch 2, **dadurch gekennzeichnet, dass** das zweite Verbindungselement (4) aus nicht-resorbierbarem Material im wesentlichen in axialer Richtung wirkende Druckkräfte und keine Biegekräfte überträgt.

## Claims

1. Endoprosthesis, in particular a hip joint prosthesis, comprising a stem and a joint part, whereby the upper stem part (1) and the lower stem part (2) are connected by means of a connection element (3), **characterized in that** the connection element (3) is manufactured from bioresorbable material.

2. Endoprosthesis according to claim 1, **characterized in that** a second connection element (4) made from non-resorbable material, for example, high-quality steel, is arranged in the region of the first connection element (3) made from bioresorbable material.

3. Endoprosthesis according to claim 2, **characterized in that** the second connection element (4) made from non-resorbable material transmits compressive forces acting substantially in the axial direction and does not transit any bending forces.

## Revendications

1. Endoprothèse, en particulier prothèse de l'articulation coxo-fémorale, constituée d'une tige et d'une partie d'articulation, la partie supérieure (1) de la tige et la partie inférieure (2) de la tige étant reliées au moyen d'un élément de jonction (3),
**caractérisée en ce que** l'élément de jonction (3) est fabriqué en un matériau biorésorbable.

2. Endoprothèse selon la revendication 1,
**caractérisée en ce qu'**un second élément de jonction (4) en un matériau non résorbable, par exemple en acier spécial, est disposé dans la zone du premier élément de jonction (3) en un matériau biorésorbable.

3. Endoprothèse selon la revendication 2,
**caractérisée en ce que** le second élément de jonction (4) en un matériau non résorbable transmet des forces de pression agissant pour l'essentiel dans la direction axiale mais aucune force de flexion.
